Europäisches Patentamt

European Patent Office

Office européen des brevets

(11) Veröffentlichungsnummer: **0 221 465**
**A2**

(12) **EUROPÄISCHE PATENTANMELDUNG**

(21) Anmeldenummer: 86114755.1

(22) Anmeldetag: 23.10.86

(51) Int. Cl.⁴: **B01F 3/12** , B01F 17/42 , A61K 7/00

(30) Priorität: 31.10.85 DE 3538751

(43) Veröffentlichungstag der Anmeldung:
13.05.87 Patentblatt 87/20

(84) Benannte Vertragsstaaten:
AT BE CH DE FR GB IT LI NL SE

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Höffkes, Horst, Dr.**
**Carlo-Schmid-Strasse 113**
**D-4000 Düsseldorf-Hellerhof(DE)**
Erfinder: **Blumenkamp, Konrad**
**Straelener Weg 65**
**D-4060 Viersen(DE)**
Erfinder: **Fach, Carola**
**Hakenfelder Strasse 8 b**
**D-1000 Berlin 20(DE)**
Erfinder: **Tüscher, Peter ·**
**Braunschweiger Strasse 70**
**D-1000 Berlin 44(DE)**
Erfinder: **Panthel, Günter, Dr.**
**Hollenberger Busch 7**
**D-5657 Haan(DE)**

(54) Verfahren zur Herstellung von Perlglanzdispersionen.

(57) Ein Verfahren zur Herstellung wäßriger Dispersionen perlglanzbildender Feststoffe besteht darin, daß man die Feststoffe, Emulgatoren und einen Teil des Wassers bei einer Temperatur von 1 bis 30 °C oberhalb der Schmelztemperatur der Feststoffe emulgiert und dieser Emulsion das restliche Wasser mit einer solchen Temperatur zufügt, daß die Dispersion eine Temperatur von 3 bis 15 °C unterhalb der Kristallisationstemperatur der perlglanzbildenden Feststoffe annimmt. Dieses Verfahren ist besonders für die kontinuierliche Fertigung geeignet.

EP 0 221 465 A2

## "Verfahren zur Herstellung von Perlglanzdispersionen"

Gegenstand der Erfindung ist ein Verfahren zur Herstellung wäßriger Dispersionen perlglanzbildender Fettstoffe.

Wäßrigen Zubereitungen von Tensiden und kosmetischen Präparaten kann man durch Einarbeitung von Substanzen, die beim Abkühlen in Form einer Dispersion feiner, perlmuttartiger Kristalle ausfallen, ein perlmuttartiges, ästhetisch ansprechendes Aussehen verleihen. Als perlglanzbildende Substanzen eignen sich vorwiegend Fettstoffe, die eine Schmelz-bzw. Kristallisationstemperatur im Bereich von 50 bis 95 °C haben, in wäßrigen Tensidzubereitungen schwer löslich sind und in solchen Zubereitungen beim Abkühlen in Form kleiner, glänzender Kristalle von gleichartigem Habitus auskristallisieren.

Da die Erzeugung solcher perlglänzender Tensidzubereitungen besondere Sorgfalt während der Abkühlung erfordert, damit die perlglanzbildenden Fettstoffe gleichmäßig auskristallisieren, ist man dazu übergegangen, sogenannte Perlglanzkonzentrate in den Handel zu bringen, das sind Perlglanzdispersionen mit einem Gehalt von ca. 5 bis 20 Gew.-% solcher perlglanzbildenden Fettstoffe, die perlglänzend auszustattenden Tensidzubereitungen ohne Erwärmung, also bei Raumtemperatur, zugesetzt werden können.

Damit wurde das Problem der Erzeugung gleichmäßig auskristallisierter Perlglanzdispersionen auf die Herstellung dieser Perlglanzkonzentrate verlagert. Die übliche Verfahrensweise besteht darin, die perlglanzbildenden Fettstoffe gegebenenfalls mit einem Emulgator über den gemeinsamen Schmelzpunkt zu erwärmen, dann das auf eine Temperatur über diesem Schmelzpunkt erwärmte Wasser zuzugeben, durch intensives Mischen eine Emulsion zu erzeugen und schließlich diese Emulsion langsam und gleichmäßig unter ständiger Bewegung, z. B. unter Rühren, unter die Kristallisationstemperatur der Perlglanzbildner abzukühlen. Die Brillanz und Feinteiligkeit des sich bildenden Perlglanzes sind bei diesem Verfahren in hohem Maße von der Abkühlgeschwindigkeit und der mechanischen Bearbeitung der Zubereitung während der Abkühlphase (Rührgeschwindigkeit, statische oder dynamische Mischaggregate) abhängig und es hat sich als schwierig erwiesen, solche Zubereitungen in gleichmäßiger Qualität herzustellen.

Es wurde nun ein Verfahren gefunden, welches die Herstellung wäßriger Dispersionen perlglanzbildender Fettstoffe von gleichbleibend brillianter Qualität und unter leicht zu beherrschenden und wenig Zeit-und Energieaufwand erfordernden Bedingungen möglich macht und das für eine kontinuierliche Betriebsweise geeignet ist.

Das erfindungsgemäße Verfahren ist dadurch gekennzeichnet, daß man

-die perlglanzbildenden Fettstoffe, gegebenenfalls Emulgatoren und eine erste Teilmenge des Wassers einzeln oder gemeinsam erhitzt und unter Bildung einer Emulsion innig vermischt, deren Temperatur um 1 bis 30 °C über der Schmelz-bzw. Kristallisationstemperatur der perlglanzbildenden Fettstoffe liegt und danach

-die zweite Teilmenge Wasser zumischt, wobei deren Temperatur so bemessen ist, daß die dabei entstehende Dispersion eine Temperatur annimmt, die um 3 bis 15 °C unterhalb der Kristallisationstemperatur der perlglanzbildenden Stoffe liegt.

Die Kristallisationstemperatur läßt sich auf einfache Weise durch Aufnahme des Temperatur-Zeit-Verlaufs bei der Abkühlung einer Schmelze dieser Stoffe ermitteln. Das Erreichen der Kristallisationstemperatur zeigt sich daran, daß diese Temperaturkurve infolge der freiwerdenden Kristallisationswärme ihre Richtung ändert. Wenn eine Mischung von perlglanzbildenden Stoffen eingesetzt wird, so ist die Kristallisationstemperatur der verwendeten Mischung zu bestimmen.

Die Ausführung der ersten Stufe des erfindungemäßen Verfahrens kann im einzelnen sehr unterschiedlich erfolgen, wenn dafür Sorge getragen wird, daß die dabei gebildete Emulsion eine Temperatur aufweist, die wenigstens 1 °C über der Schmelz-bzw. Kristallisationstemperatur der perlglanzbildenden Stoffe liegt, so daß diese Stoffe in flüssiger Form emulgiert werden.

Die perlglanzbildenden Fettstoffe, die gegebenenfalls verwendeten Emulgatoren und die erste Teilmenge des Wassers können z. B. separat erhitzt und dann vereinigt und emulgiert werden. Man kann aber auch das Wasser und einen darin gelösten Emulgator, z. B. in Form einer wäßrigen Tensidlösung, erhitzen und mit dem separat erhitzten perlglanzbildenden Fettstoff vereinigen und emulgieren. Schließlich kann·man auch den perlglanzbildenden Fettstoff, den Emulgator und die erste Teilmenge des Wassers vereinigen und gemeinsam auf die für die Emulgierung erforderliche Temperatur von 1 bis 30 °C über der Kristallisationstemperatur des Perlglanzbildners erhitzen. Die erste Teilmenge des Wassers sollte mindestens 10 Gew.-% des gesamten Wasseranteils der Dispersion und die zweite Teilmenge des Wassers mindestens 25 Gew.-% des gesamten Wasseranteils der Dispersion ausmachen. Bei diskontinuierlicher Herstellung ist es bevorzugt, die Emulsion mit einer relativ geringen ersten Wassermenge von 10 bis 30 Gew.-% des gesamten

2

Wasseranteils und bei einer relativ hohen Temperatur von etwa 10 bis 30 °C oberhalb der Kristallisationstemperatur herzustellen. Bei kontinuierlicher Arbeitsweise hat es sich als vorteilhafter erwiesen, die Emulsion mit einer relativ höheren ersten Wassermenge von 30 bis 70 Gew.-% des gesamten Wasseranteils bei einer Temperatur herzustellen, die nur etwa 1 bis 10 °C oberhalb der Kristallisationstemperatur des Perlglanzbildners liegt.

Die innige Vermischung von Perlglanzbildner, Emulgator und Wasser kann im einfachsten Falle unter Verwendung hochtouriger Rührwerke oder Turbinen durchgeführt werden, es eignen sich aber auch andere für die Herstellung von Emulsionen geeignete Mischaggregate entweder statischer oder dynamischer Arbeitsweise. Bevorzugt werden dynamische Mischaggregate, wie der im Beispiel verwendete Pentax ® - Mischer verwendet.

Die Zumischung der zweiten Teilmenge des für die Dispersion erforderlichen Wassers kann ebenfalls unter Verwendung üblicher statischer oder dynamischer Mischeinrichtungen erfolgen. Im einfachsten Falle kann bei diskontinuierlicher Herstellung das gleiche Mischaggregat wie für die Herstellung der Emulsion verwendet werden. Bei kontinuierlicher Herstellung werden bevorzugt statische Mischaggregate verwendet.

Besonders wichtig ist aber bei der Zumischung der zweiten Teilmenge des Wassers die richtige Wahl der Wassertemperatur. Es kommt dabei darauf an, daß durch die von der zugemischten zweiten Teilmenge des Wassers aufgenommen Wärme die Temperatur der sich bildenden Dispersion auf 3 bis 15 °C unter die Kristallisationstemperatur der perlglanzbildenden Fettstoffe abgesenkt wird.

Eine grobe Abschätzung der dafür erforderlichen Wassertemperatur kann anhand der calorischen Daten (insbesondere der spezifischen Wärme) des Wassers und der verwendeten Fettstoffe leicht vorgenommen werden. Die genaue Einstellung der erforderlichen Temperaturen unter Berücksichtigung der durch die Apparatur bedingten Wärmeverluste und der in den Mischern entstehenden Reibungswärme läßt sich ohne Schwierigkeiten experimentell ermitteln.

Die Temperatur der zweiten Teilmenge des Wassers sollte aber möglichst nicht mehr als 30 °C, bevorzugt nur 5 bis 20 °C unterhalb der Kristallisationstempertur der perlglanzbildenden Stoffe liegen.

Das erfindungsgemäße Verfahren eignet sich besonders gut für eine kontinuierliche Herstellung. Die kontinuierliche Arbeitsweise zeichnet sich bevorzugt dadurch aus, daß

-die perlglanzbildenden Fettstoffe, die Emulgatoren und die erste Teilmenge des Wassers getrennt aufgeheizt und einem üblichen, bevorzugt dynamischen, Mischaggregat zugeführt und emulgiert werden, wobei die Temperatur der Komponenten so gewählt wird, daß die Emulsion ohne zusätzliche Wärmezufuhr eine Temperatur annimmt, die um 1 bis 30 °C über der Schmelz-bzw. Kristallisationstemperatur der perlglanzbidlenden Stoffe liegt,

-die zweite Teilmenge des Wassers auf die berechnete Temperatur erwärmt und in die gebildete Emulsion eingeleitet wird, wobei die Temperatur des Wassers so berechnet wird, daß die Temperatur der sich bildenden Dispersion um 3 bis 15 °C unterhalb der Kristallisationstemperatur der perlglanzbildenden Fettstoffe liegt und

-die gebildete Dispersion einem oder mehreren üblichen, bevorzugt statischen Mischaggregaten zur Homogenisierung zugeführt wird.

Für die Ausführung des erfindungsgemäßen Verfahrens sind alle bekannten perlglanzbildenden Fettstoffe und Gemische solcher Fettstoffe geeignet, die eine Kristallisationstemperatur im Bereich von 50 bis 95 °C aufweisen. Besonders geeignet sind Ester der allgemeinen Formel $R^1-(OC_nH_{2n})-OR^2$, in der $R^1$ eine lineare Acylgruppe mit 16 bis 22 C-Atomen, $R^2$ Wasserstoff oder eine Acylgruppe $R^1$, n = 2 oder 3 und x eine Zahl von 1 bis 4 ist. Beispiele solcher besonders geeigneter Fettstoffe sind z. B. Ethylenglycoldistearat und Triethylenglycoldistearat.

Besonders geeignet sind Gemische solcher Ester mit Monoethanolamiden von $C_{12}$-$C_{18}$-Fettsäuren, z. B. Kokosfettsäure-$C_{12}$-$C_{18}$-monoethanolamid.

Weitere Produkte, die als perlglanzbildende Fettstoffe oder als Komponenten solcher perlglanzbildender Fettstoffe verwendet werden, sind z. B. Glycerin-mono-und distearat, Pentaerythrit-mono-und distearat, Sorbitan-mono-und distearat und $\beta$-Ketosulfone der allgemeinen Formel $R^3-CHR^4-SO_2-CH_2-R^5$, in der $R^3$ eine Acylgruppe mit 12 bis 22 C-Atomen, $R^4$ und $R^5$ Wasserstoffatome oder zusammen eine Ethylengruppe darstellen, die mit der zwischen $R^4$ und $R^5$ liegenden Gruppe einen Tetrahydrothiophendioxidring bildet, z. B. 1-(Methylsulfonyl)-nonadecan-2-on oder 2-Stearoyl-tetrahydrothiophen-1,1-dioxid. Weiterhin eignen sich auch Dialkylsulfide bzw. Dialkylsulfoxide wie z. B. Di-n-dodecylsulfoxid und Di-n-hexadecylsulfid.

3

Als Emulgatoren bzw. Dispergierhilfsmittel können z. B. anionische und/oder nichtionogene Emulgatoren eingesetzt werden. Als anionische Emulgatoren eignen sich z. B. Fettalkoholsulfate und Fettalkoholpolyglycolethersulfate der allgemeinen Formel $R^5$-$O(CH_2$-$CH_2$-$O)_y$-$SO_3^{(-)}$, in der $R^5$ eine lineare Alkylgruppe mit 10 bis 18 C-Atomen, y = 0 oder eine Zahl von 1 bis 20 darstellt, in Form ihrer Natrium-, Kalium-, Magnesium-, Ammonium-, Mono-, Di-oder Triethanolammoniumsalze. Solche anionischen Emulgatoren werden bevorzugt in Form wäßriger Lösungen eingesetzt oder der ersten Teilmenge des Wassers zugesetzt und mit diesem gemeinsam aufgeheizt.

Bevorzugt werden aber nichtionische Emulgatoren zur Stabilisierung der Perlglanzdispersionen verwendet. Unter den zahlreichen nichtionogenen Tensiden eignen sich besonders die Anlagerungsprodukte von 3 bis 20 Mol Ethylenoxid an gesättigte Fettalkohole mit 12 bis 22 C-Atomen. Diese nichtionogenen Emulgatoren können auch separat aufgeheizt oder mit dem perlglanzbildenden Fettstoffen gemischt und mit diesen gemeinsam aufgeschmolzen werden.

Der fertigen Perlglanzdispersion können Konservierungsstoffe wie z. B. Formaldehyd, p-Hydroxybenzoesäureester, Sorbinsäure, Chloracetamid, Natriumbenzoat, 5-Brom-5-nitro-1,3-dioxan oder andere bekannte Konservierungsmittel zugesetzt werden, um die bakterielle Zersetzung der Fettstoffe zu hemmen.

Die folgenden Beispiele sollen den Erfindungsgegenstand näher erläutern, ohne ihn hierauf zu beschränken:

## Beispiele

Als perlglanzbildender Fettstoff wurde eingesetzt ein Gemisch aus
80 g Ethylenglycoldistearat (CTFA-Bezeichnung: Glycol Distearate) und
40 g Kokosfettsäure($C_{12}$-$C_{18}$)monoethanolamid.
Dieses perlglanzbildende Gemisch zeigte eine Kristallisationstemperatur bei 58 °C.

Als Emulgator wurde verwendet ein Anlagerungsprodukt von 4 Mol Ethylenoxid an ein $C_{12}/C_{14}$-Fettalkoholgemisch (70 : 30) (Dehydol ® LS4).

### 1. Diskontinuierliche Herstellung

Eine Mischung aus 80 kg Ethylenglycoldistearat, 40 kg Kokosfettsäure-$C_{12}$-$C_{18}$-monoethanolamid und 50 kg Dehydol ® LS4 wurde auf 75 °C erhitzt. Dann wurden 80 kg Wasser auf 75 °C erwärmt und zugegeben und durch Homogenisierung mit einer Turbine eine Emulsion gebildet. Dann wurde die restliche Wassermenge von 750 kg auf 25 °C erwärmt und unter Rühren zu der Emulsion gegeben. Es bildete sich eine Dispersion, deren Temperatur 47 °C betrug und die nach wenigen Minuten ein brillantes, perlglänzendes Aussehen annahm. Diese Dispersion konnte ohne weitere Behandlung in das Vorratsgefäß gefüllt werden und war für die sofortige Verwendung geeignet.

### 2. Kontinuierliche Herstellung

In einer kontinuierlichen Anlage gemäß Abb. 1 wurden
-ein Gemisch
aus 80 Gewichtsteilen Ethylenglycoldistearat und 40 Gewichtsteilen Kokosfettsäure-$C_{12}$-$C_{18}$-monoethanolamid mit einer Temperatur von 77 °C (1)
-415 Gewichtsteilen Wasser mit einer Temperatur von 55 °C (2)
-50 Gewichtsteilen Dehydol ® LT 4 (3)
pro Zeiteinheit einem Pentax-Mischer (P) zugeführt. Ohne weitere Wärmezu-oder abfuhr wurden in die, dem Pentax-Mischer verlassende Emulsion (T1 = 60 °) 415 Gewichtsteile Wasser mit einer Temperatur von 50 °C (4) pro Zeiteinheit mit Hilfe von hintereinander angeordneten statischen Mischaggregaten, einem N-Form-Mischer (N) und einem Doppelventil-Mischer (D), eingemischt. Die dabei gebildete Dispersion hatte eine Temperatur von 53 °C (T2) und wurde in weiteren hintereinander angeordneten statischen Mischaggregaten (N und D) weiter homogenisiert.

Erläuterungen:

1 Gemisch der perlglanzbildenden Fettstoffe, Kristallisationstemperatur 58 °C
2 Erste Teilmenge Wasser
3 Emulgator
4 Zweite Teilmenge Wasser
P Pentax-Mischer (dynamisches Mischaggregat)
N N-Form-Mischer (statisches Mischaggregat)
D Doppelventil-Mischer (statisches Mischaggregat)
T1 : 60 °C
T2 : 53 °C

**Ansprüche**

1. Verfahren zur Herstellung wäßriger Dispersionen perlglanzbildender Fettstoffe, dadurch gekennzeichnet, daß man
-die perlglanzbildenden Fettstoffe, Emulgatoren und eine erste Teilmenge des Wassers einzeln oder gemeinsam erhitzt und unter Bildung einer Emulsion innig vermischt, deren Temperatur um 1 bis 30 °C über der Schmelz-bzw. Kristallisationstemperatur der perlglanzbildenden Fettstoffe liegt und danach
-die zweite Teilmenge des Wassers zumischt, wobei deren Temperatur so bemessen ist, daß die dabei entstehende Dispersion eine Temperatur annimmt, die um 3 bis 15 °C unterhalb der Kristallisationstemperatur der perlglanzbildenden Fettstoffe liegt.

2. Verfahren nach Anspruch 1, dadurch gekennzeichnet, daß die erste Teilmenge des Wassers mindestens 10 Gew.-% des Wasseranteils der Dispersion und die zweite Teilmenge des Wassers mindestens 25 Gew.-% des Wasseranteils der Dispersion ausmacht.

3. Verfahren nach den Ansprüchen 1 oder 2, dadurch gekennzeichnet, daß die Temperatur der zweiten Teilmenge des Wassers nicht mehr als 30 °C, bevorzugt 5 bis 20 °C unterhalb der Kristallisationstemperatur der perlglanzbildenden Fettstoffe liegt.

4. Verfahren nach den Ansprüchen 1 bis 3, dadurch gekennzeichnet, daß die Herstellung der Dispersion kontinuierlich durchgeführt wird, wobei
-die perlglanzbildenden Fettstoffe, die Emulgatoren und die erste Teilmenge des Wassers getrennt aufgeheizt und einem üblichen, bevorzugt dynamischen, Mischaggregat zugeführt und emulgiert werden, wobei die Temperatur der Komponenten so gewählt wird, daß die Emulsion ohne zusätzliche Wärmezufuhr eine Temperatur annimmt, die um 1 bis 30 °C über der Schmelz-bzw. Kristallisationstemperatur der perlglanzbildenden Fettstoffe liegt,
-die zweite Teilmenge des Wassers auf die berechnete Temperatur erwärmt und in die gebildete Emulsion eingeleitet wird, wobei die Temperatur des Wassers so berechnet wird, daß die Temperatur der sich bildenden Dispersion um 3 bis 15 °C unterhalb der Kristallisationstemperatur der perlglanzbildenden Stoffe liegt und
-die gebildete Dispersion einem oder mehreren üblichen, bevorzugt statischen Mischaggregaten zur Homogenisierung zugeführt wird.

5. Verfahren nach den Ansprüchen 1 bis 4, dadurch gekennzeichnet, daß als perlglanzbildende Fettstoffe Ester der allgemeinen Formel $R^1-(OC_nH_{2n})-OR^2$, in der $R^1$ eine lineare Fettacylgruppe mit 16 bis 22 C-Atomen, $R^2$ Wasserstoff oder eine Acylgruppe $R^1$, n = 2 oder 3 und x eine Zahl von 1 bis 4 ist, oder Gemische solcher Ester mit Monoethanolamiden von $C_{12}-C_{18}$-Fettsäuren eingesetzt werden.

6. Verfahren nach den Ansprüchen 1 bis 5, dadurch gekennzeichnet, daß als Emulgatoren nichtionogene Anlagerungsprodukte von 3 bis 20 Mol Ethylenoxid an gesättigte Fettalkohole mit 12 bis 22 C-Atomen eingesetzt werden.